(19) Europäisches Patentamt
European Patent Office
Office européen des brevets

(11) Publication number: **0 005 492**
**B1**

(12) **EUROPEAN PATENT SPECIFICATION**

(45) Date of publication of patent specification: 23.02.83

(21) Application number: 79101379.0

(22) Date of filing: 07.05.79

(51) Int. Cl.³: C 07 C 29/15, C 07 C 31/04, C 07 C 31/08, B 01 J 23/76, C 10 L 1/02

(54) **Preparation of alcohols from synthesis gas.**

(30) Priority: 15.05.78 US 905703

(43) Date of publication of application: 28.11.79 Bulletin 79/24

(45) Publication of the grant of the patent: 23.02.83 Bulletin 83/8

(84) Designated Contracting States: AT BE CH DE FR GB IT LU NL SE

(56) References cited:
DE - C - 625 757
FR - A - 705 590
FR - A - 1 011 967
FR - A - 1 074 045
GB - A - 707 972
GB - A - 1 159 035
NL - C - 16 114

(73) Proprietor: THE STANDARD OIL COMPANY
Midland Building
Cleveland, Ohio 44115 (US)

(72) Inventor: Hardman, Harley F.
4989 Delevan Drive
Lyndhurst, Ohio 44124 (US)
Inventor: Beach, Ronald I.
15539 Wenhaven Drive
Chagrin Falls, Ohio 44022 (US)

(74) Representative: Redies, Bernd, Dr. rer. nat. et al,
Redies, Redies, Türk & Gille Brucknerstrasse 20
D-4000 Düsseldorf 13 (DE)

EP 0 005 492 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Preparation of alcohols from synthesis gas

The present invention relates to a new process for making alcohol mixtures preferably containing no more than 85% methanol and to gasoline compositions containing such alcohol mixtures.

It is well known to incorporate methanol into gasoline for the purpose of increasing the amount of useful fuel obtained. Unfortunately, the presence of a small amount of water in a methanol/gasoline mixture causes separation of the methanol and gasoline phases. In order to overcome this problem, higher alcohols can be added to methanol/gasoline mixtures, such compositions being able to tolerate a much greater amount of water than a simple methanol/gasoline mixture. See U.S.—A—3,832,149, U.S.—A—3,822,119 and U.S.—A—2,128,910. Higher alcohols can also be used alone. See U.S.—A—2,078,736.

Although it has been known for many years that alcohols can be incorporated into gasolines, most gasolines made at this time contain no alcohols, and this is due primarily to economic reasons.

It has also been long known to prepare alcohols by catalytically reacting carbon monoxide and hydrogen together using catalysts which may contain copper and thorium. See, for example, U.S.—A—1,707,331, U.S.—A—2,061,470, U.S.—A—2,500,913, U.S.—A—1,741,307, U.S.—A—1,625,929 and U.S.—A—1,831,179. Such processes, however, normally require extremely high reaction temperatures and pressures and hence have not been employed commercially to any significant extent.

Accordingly, it is an object of the present invention to provide a simple, straightforward and very inexpensive process for producing alcohol mixtures for incorporating into gasoline.

In addition, it is a further object of the present invention to provide a novel alcohol mixture and in addition a gasoline composition containing this novel alcohol mixture.

These and other objects are accomplished by the present invention in accordance with which a mixture of methanol and higher alcohols is produced by contacting synthesis gas with a novel copper/thorium/alkali metal oxide catalyst. Alcohol compositions produced by this procedure contain a unique distribution of alcohols having 2 to 6 carbon atoms and normally not more than 85% methanol by weight. These compositions can be directly admixed with gasoline to provide a new gasoline composition containing a mixed alcohol system which will not separate from the remainder of the gasoline even in the presence of significant amounts of water.

Thus, the present invention provides a unique process for producing alcohol mixtures, the inventive process comprising contacting a gaseous reactant containing carbon monoxide and hydrogen with an oxide complex catalyst described by the formula

$$Cu_aThM_bA_cO_x$$

wherein

M is one or more of Ca, Mo, Rh, Mn, Pt, Ce, Cr, Zn, Al, Ti, La, V, U, Ru, Re and Pd;

A is an alkali metal; and

wherein

a is 0.5 to 2.5;

b is 0.01 to 1.0;

c is 0.05 to 0.9; and

x is a number such that the valence requirements of the other elements for oxygen is satisfied.

In addition, the present invention further provides a novel alcohol composition suitable for admixing with gasoline, the alcohol composition being composed of $C_2$ and higher alcohols having an alcohol distribution as follows:

| | |
|---|---|
| $C_2$ | 4—25% |
| $C_3$ | 0.1—25%, preferably 9—25% |
| $C_4$ | 0.5—70%, preferably 40—70% |
| $C_5$ | 0.1—12% |
| $C_6$ | 0.1—10% |
| $C_6$ | 0.1—10% |

the percents being based on the weight of the total amount of alcohols in the mixture having two or more carbon atoms. Normally, this alcohol composition will also contain methanol, usually in amounts of 40 to 85% although the methanol content may be 92% or more.

Furthermore, the present invention also provides a novel alcohol/gasoline composition comprising a homogenous solution of gasoline and the novel alcohol composition of the present invention.

## Reactants

The material being reacted in accordance with the present invention to form alcohols is preferably synthesis gas. As is well known, synthesis gas is composed basically of a mixture of hydrogen and carbon monoxide in which the $H_2/CO$ ratio is from 0.6 to 6, more normally between 1 and 2. It is normally derived by heating coke in the presence of air and then steam. Alternately, it can also be produced by partial combustion of coal, natural gas or petroleum hydrocarbons. It is sometimes referred to as "water gas." Synthesis gas normally contains a very low amount of sulfur compounds. It also may contain small amounts of carbon dioxide, nitrogen and other inerts.

Although synthesis gas is a preferred reactant, any other gas composed primarily of hydrogen and carbon monoxide and having an

$H_2/CO$ ratio of 0.6 to 6 can be employed. The gaseous reactant should contain as little sulfur compounds as possible since sulfur is a known poison for copper containing catalysts when used in conventional methanol synthesis. Preferably the gaseous reactant is essentially sulfur-free.

Reaction mode and conditions

The inventive process is carried out by contacting the gaseous reactant with the inventive catalyst as described below in a suitable reactor. The reaction can be carried out either in fluid-bed mode or fixed-bed mode, continuously or in batch operation.

The contact time of the reactants with the products is not critical but should be below 200 seconds and preferably between 35 and 135 seconds.

The reaction pressure should normally be between 34—103 bar (500—1500 psi) and is preferably between 51—69 bar (750—1000 psi). Although there is no real upper limit to the reaction pressure, pressures higher than 103 bar (1500 psi) are normally not employed because of the high expense involved. Also, pressures as low as 17 bar (250 psi) can be employed, although it is preferable to operate at at least 34 bar (500 psi) because formation of alcohols is favored at higher pressures.

The reaction temperature should be maintained between 200—425°C, preferably 250—350°C, and most preferably 280—330°C. The reaction temperature, like the reaction pressure, is not particularly critical, although a marked decrease in conversion rates will be obtained if temperatures and pressures lower than 17 bar (250 psi) and 200°C are employed. Also, the amount of methanol in the reaction product increases with practically all catalysts when the reaction temperature drops below 200°C. It will be noted that temperatures and pressures employed in this process are quite a bit lower than temperatures and pressures necessary in prior art processes for producing mixed alcohols from synthesis gas.

Catalyst

The catalyst employed in the inventive process is believed to be an oxide complex and can be described by the empirical formula:

$$Cu_aThM_bA_cO_x$$

wherein

M is one or more of Ca, Mo, Rh, Mn, Pt, Ce, Cr, Zn, Al, Ti, La, V, U, Ru, Re or Pd, preferably Cr, Zn, Al, Ti, La, V or Pd;

A is an alkali metal, preferably Na; and wherein

a is 0.5 to 2.5, preferably 1.3 to 1.7;

b is 0.01 to 1.0, preferably 0.01 to 0.6;

c is 0.05 to 0.9, preferably 0.225 to 0.55; and

x is a number such that the valence requirements of the other elements for oxygen is satisfied.

These catalysts can be prepared by a procedure involving adding an alkali metal carbonate to an aqueous solution containing decomposable salts of thorium, copper and the "M" element, to form a precipitate heating the precipitate in its mother liquor for a suitable time, neutralizing the precipitate, drying the precipitate and thereafter calcining and then reducing the precipitate.

More specifically, the catalysts of the present invention can be prepared by the following procedure:

(1) A solution preferably aqueous, containing decomposable salts of thorium, copper and the "M" element is first formed. Nitrates are preferably used as the decomposable salts, although salts having other decomposable anions such as acetates, propionates, benzoates, acetyl acetonates, naphthenates, chlorides and the like can be employed.

(2) To the aqueous salt solution is added alkali metal carbonate, preferably also in the form of an aqueous solution. During this addition, the admixture is preferably maintained at an elevated temperature, e.g. 80—95°C. As the alkali metal carbonate is added to the salt solution, carbon dioxide is evolved and a sky-blue precipitate whose exact nature is unknown begins to form. Addition of alkali metal carbonate is continued while maintaining the temperature of the system preferably between 80—95°C until the pH of the system, which is initially at about 1, increases to at least 7.5, preferably at least 8, most preferably 9.3—10. At this time a gelatinous sky-blue precipitate in significant amount has been formed. An alkali metal hydroxide can be used in place of the carbonate, although the carbonate is preferred because it gives better results.

(3) This gelatinous precipitate is then allowed to digest in its mother liquor while the temperature of the system is maintained at its elevated value, preferably 80—95°C. During this time, it will be noted that the color of the precipitate undergoes a significant change from sky-blue through green through brown to nearly black.

(4) When no more color change occurs, an acid, preferably nitric acid, is added to bring the pH to about neutral, preferably 6.8—7, which is believed to cause precipitation of any dissolved Cu remaining in the mother liquor. The precipitate is then filtered and washed with water until the alkali metal content of the finished catalyst falls to the desired value. This can be easily determined by trial and error.

(5) The precipitate is then dried at a convenient temperature, for example 120°C, and then calcined for a time and at a temperature sufficient to drive out the remaining water in the precipitate and decompose decomposable ions remaining in the precipitate, such as nitrates and carbonates. Temperatures of 250—500°C,

preferably 350—450°C for periods of 30 minutes to about 5 hours have been found sufficient for this purpose.

(6) Since the catalyst of the present invention is used in a reducing atmosphere, it is preferable to reduce the calcined precipitate prior to use in the inventive process. Prereduction of the catalyst, however, is not necessary, since the catalyst will automatically undergo reduction to an equilibrium value in use, although the activity of the catalyst may not be as great as when a proper prereduction procedure is carried out. In this regard, it is believed that heating of the calcined precipitate to higher temperatures will cause significant reduction in the activity of the ultimate catalyst produced due to sintering of particles of the calcined precipitate. Since a very exothermic reaction may occur when a reducing gas is contacted with the calcined precipitate, it is preferable to subject the calcined precipitate to a controlled reduction procedure in order to avoid heating the calcined precipitate to above 300°C. Therefore, it is preferable to carry out a controlled reduction of the calcined precipitate in the following manner, although any other satisfactory technique could be employed as well.

The calcined precipitate is first heated under inert atmosphere preferably in the alternate reaction vessel to a temperature of about 200°C. Next, an inert gas containing a low concentration (e.g. about 5%) of a reducing gas, preferably $H_2$ although a mixture of $H_2$ and CO can be employed, is admitted to the gas surrounding the catalyst for initial reduction of the metal and concomitant heat evolution. Thereafter, the concentration of the reducing gas is slowly increased to 100%, care being taken to keep the rate of reducing gas applied low enough to prevent the temperature of the catalyst from exceeding a value at which significant sintering occurs, usually 300°C. Once 100% reducing gas is reached, the pressure in the reaction vessel is slowly increased to the desired reaction pressure, the catalyst now being ready to receive reactant.

The catalysts of the present invention can be used alone or supported on various inert supports such as silica, alpha-alumina, Alundum, mullite and the like. These materials are preferably low surface area supports and can be added to the catalyst during its preparation (i.e. after the sky-blue precipitate is first formed) or after the preparation of the catalyst in conventional manners.

Product

The composition produced by the inventive process is a mixture of alcohols containing methanol predominantly as well as significant amount of higher alcohols usually having 2—8 carbon atoms. Normally, the alcohol product of the present invention will contain 40—85% methanol, although higher amounts of methanol may be included in the product if reaction temperature is too low, if the catalyst is calcined at too high a temperature or if the catalyst contains additional elements fostering the generation of higher amounts of methanol than normal.

In this regard, it has been found that the amount of methanol in the product alcohol mixture will usually exceed 85% if the M element in the catalyst is chosen as Ca, Mo, Rh, Mn, Pt, Re, Ru or in some instances Ce. For this reason, Cr, Zn, Al, Ti, La, V and Pd are preferred as the M element. In any event, an alcohol mixture produced by the inventive process and containing over 85% methanol can also be directly added to gasoline as is. It is preferable, however, to distill off excess methanol so that the methanol content of the alcohol mixture is no more than 85%

The portion of the alcohol product other than methanol is a mixture composed substantially completely of 2—6 carbon atom alcohols. This $C_2$—$C_6$ alcohol mixture has an alcohol distribution scheme as given in the following Table I.

TABLE I

| | |
|---|---|
| $C_2$ | 4—25% |
| $C_3$ | 0.1—25%, preferably 9—25% |
| $C_4$ | 0.5—70%, preferably 40—70% |
| $C_5$ | 0.1—12% |
| $C_6$ | 0.1—10% |
| $C_6$ | 0.1—10% |

the percents being based on the weight of the total amount of alcohols in the product having 2 or more carbon atoms. These alcohols are composed almost completely of isoalcohols and normal alcohols with the iso/normal ratio being 0.7/2. Substantially no tertiary alcohols are present.

Gasoline

The product alcohol mixtures of the present invention (whether containing more or less than 85% methanol) are useful in expanding gasoline. They can be mixed with gasoline in any amount, and when present in amounts of less than 25% no significant effect on the operation of an internal combustion engine containing the gasoline/alcohol mixture will be noticed. Furthermore, the mixed alcohol products of the present invention can be mixed with any type of gasoline be it substantially all paraffinic such as alkylate or highly aromatic. Moreover, if the product alcohol mixture employed has no more than 85% methanol, the resultant gasoline will be able to tolerate significant amounts of water without phase separation.

Specific embodiments

In order to more thoroughly describe the present invention, the following specific examples are provided.

Catalyst preparation

Example 1 $Cu_{1.5}ThPd_{0.05}Na_yO_x$

To 660 cc of distilled water were added 60.66 grams of $Cu(NO_3)_2 \cdot 2\frac{1}{2}H_2O$ and 95.99 grams of $Th(NO_3)_4 \cdot 4H_2O$. The nitrates were dissolved by warming with stirring, and then 1.54 grams of $PdCl_2$ were dissolved in the nitrate solution. The nitrate solution was heated to 90°C, and with high speed stirring there was added a hot (90—95°C) solution of 73.2 grams of $Na_2CO_3$ in 800 cc of distilled $H_2O$. Using a pH meter, sufficient additional $Na_2CO_3$ was added to bring the pH of the slurry to 9.5. Heating was continued until the color change to dark brown was complete, about half of the water being evaporated. The slurry was then cooled to room temperature, and the pH adjusted to 7.0 with a 20% $HNO_3$ solution. The slurry was filtered using vacuum, and sucked as dry as possible. The filter cake was washed three times by re-slurrying in 800 cc of distilled $H_2O$ at 60—70°C, and refiltering. It was then dried approximately 16 hours at 120°C, and calcined 3 hours at 275°C and 16 hours at 350°C in air. The sodium content of the finished catalyst was 1.6 weight %.

Example 2 $Cu_{1.5}ThCr_{0.3}Na_yO_x$

The procedure of Example 1 was followed except that 20.9 grams of $Cr(NO_3)_3 \cdot 9H_2O$ rather than 1.54 grams $PdCl_2$ was dissolved in the copper-thorium nitrate solution. The final catalyst contained 0.55 weight % Na.

Example 3 $Cu_{1.5}ThLa_{0.15}Na_yO_x$

The procedure of Example 1 was followed except that 11.3 grams of $La(NO_3)_3 \cdot 6H_2O$ rather than the $PdCl_2$ was added to the copper-thorium nitrate solution. In addition the final catalyst contained 1.45 weight % Na.

Example 4 $Cu_{1.5}ThCe_{0.30}Na_yO_x$

The procedure of Example 1 was followed except that 22.6 grams of $Ce(NO_3)_3 \cdot 6H_2O$ rather than the $PdCl_2$ was added to the copper-thorium nitrate solution. In addition, the catalyst contained 0.9 weight % Na.

Example 5 $Cu_{1.5}ThTi_{0.15}Na_yO_x$

The procedure of Example 1 was followed except that in place of the $PdCl_2$ 2.1 grams of $TiO_2$ were suspended in the copper-thorium nitrate solution before precipitation. The catalyst contained 0.68% Na.

Example 6 $Cu_{1.5}ThZn_{0.15}Na_yO_x$

78 grams of $Cu(C_2H_3O_2)_2 \cdot H_2O$, 144 grams of $Th(NO_3)_4 \cdot 4H_2O$ and 4.78 grams of ZnO in 1 liter of water were heated to 90°C. Precipitation was done with a hot solution of 117.3 grams $Na_2CO_3$ in 1.2 liters of $H_2O$. The pH was adjusted to 9.8 with additional $Na_2CO_3$. The balance of the preparation was accomplished in the same manner as set forth above in connection with Example 1.

Example 7 $Cu_{1.5}ThAl_{0.15}Na_yO_x$

In 1 liter of distilled water were dissolved 94.5 grams $Cu(NO_3)_2 \cdot 3H_2O$, 144 grams of $Th(NO_3)_4 \cdot 4H_2O$ and 22.0 grams of $Al(NO_3)_3 \cdot 9H_2O$ and the solution was heated to 90°C. This was added to a solution of 170.4 grams of $Na_2CO_3$ in 1.2 liters of distilled $H_2O$ and heated to 90—95°C. The pH of the slurry was adjusted to 9.5 with additional $Na_2CO_3$. The remainder of the preparation was carried out in the same manner as set forth above in connection with Example 1. The catalyst contained 1.47 weight % Na.

Example 8 $Cu_{1.5}ThPd_{0.05}Na_yO_x$

The procedure of Example 1 was repeated except that 1.95 grams of $Pd(C_3H_8O_2)_2$ were employed in place of the $PdCl_2$ of Example 1 Also, the sodium content of the catalyst obtained was 1.28 weight %.

Example 9 $CuThU_{0.2}Na_yO_x$

The procedure of Example 1 was repeated using 58.9 grams $Cu(NO_3)_2 \cdot 3H_2O$, 134.6 grams $Th(NO_3)_4 \cdot 4H_2O$ and 24.5 grams $(UO_2)(NO_3)_2 \cdot 6H_2O$ dissolved in 600 cc of water to form the aqueous salt solution. The catalyst contained 1.6% Na.

Example 10 $Cu_{1.5}ThCr_{0.15}Na_yO_x$

The procedure of Example 1 was repeated except that 94.5 g $Cu(NO_3)_2 \cdot 3H_2O$, 144 g $Th(NO_3)_4 \cdot 4H_2O$ and 23.5 g $Cr(NO_3)_3 \cdot 9H_2O$ in distilled water were used and in addition 119.1 gm $Na_2CO_3$ in 1 liter of water was used. The catalyst obtained contained 1.0% Na.

Example 11 $Cu_{1.5}ThCe_{0.15}Na_yO_x$

The procedure of Example 10 was repeated except that 25.5 gm Ce $(NO_3)_3 \cdot 6H_2O$ and 120 gm $Na_2CO_3$ in 2 liters water were used. The catalyst contained 1.21% Na.

Alcohol production

Examples 12 to 22 and Comparative Examples A and B

A series of experiments was run in order to determine the catalytic activity of the various catalysts prepared in Examples 1—11 in the production of alcohol mixtures. In each experiment, 40 cc of catalyst was charged into a fixed-bed reactor and contacted with an $H_2/CO$ mixture having an $H_2/CO$ ratio of approximately 1/1. The processes were carried out at a temperature of 288°C, a pressure of 51 bar (750 psig) and a contact time of 52 seconds. The results obtained are tabulated in the following Tables II and III.

Examples 23 to 33

Additional experiments were conducted using the same conditions as in Examples 12 to 22. The catalysts used in these experiments are described by the general formula.

$$Cu_{1.5}ThM_bNaO_x$$

with the $M_b$ member of each catalyst and the results obtained being set forth in the following Table IV.

TABLE II

Percent yield of methanol and higher alcohols

| Example | Catalyst composition | % Yield* Alcohols | % Yield* $CO_2$ | Product Composition (Wt. %) MeOH | Product Composition (Wt. %) $C_{2+}$ Alcohols | $C_1/C_{2+}$ Alcohol Ratio |
|---|---|---|---|---|---|---|
| 12 | $Cu_{1.5}ThPd_{0.05}Nn_yO_x$ | 19.2 | 11.3 | 67.1 | 30.7 | 2.2 |
| 13 | $Cu_{1.5}ThCr_{0.3}Na_yO_x$ | 16.5 | 10.9 | 83.5 | 15.0 | 5.6 |
| 14 | $Cu_{1.5}ThLa_{0.15}Na_yO_x$ | 15.9 | 10.9 | 83.4 | 15.6 | 5.3 |
| 15 | $Cu_{1.5}ThCe_{0.3}Na_yO_x$ | 16.1 | 13.3 | 75.8 | 23.1 | 3.3 |
| 16 | $Cu_{1.5}ThTi_{0.15}Na_yO_x$ | 15.3 | 11.0 | 84.4 | 15.3 | 5.5 |
| 17 | $Cu_{1.5}ThZn_{0.15}Na_yO_x$ | 17.1 | 9.0 | 77.7 | 18.1 | 4.3 |
| 18 | $Cu_{1.5}ThAl_{0.15}Na_yO_x$ | 13.9 | 9.1 | 84.0 | 15.1 | 5.6 |
| 19 | $Cu_{1.5}ThPd_{0.05}Na_yO_x$ | 19.5 | 11.5 | 64.6 | 33.0 | 2.0 |
| 20 | $Cu_{1.0}ThU_{0.2}Na_yO_x$ | 4.91 | 3.34 | 91.5 | 8.5 | |
| 21 | $Cu_{1.5}ThCr_{0.15}Na_yO_x$ | 15.8 | 8.6 | 78.8 | 21.1 | 3.7 |
| 22 | $Cu_{1.5}ThCe_{0.15}Na_yO_x$ | 17.4 | 9.8 | 77.8 | 21.1 | 3.7 |
| A | $Cu_{0.51}ZnAl_{0.760}O_x$ (commercial MeOH catalyst) | 14.4 | 7.3 | 93.5 | 5.9 | 15.8 |
| B | $Cu_{0.51}ZnAl_{0.760}$+2.5% Na | 15.9 | 5.8 | 93.4 | 6.3 | 14.8 |

$$*\frac{\text{Moles product produced}}{\text{Moles CO fed}}$$

TABLE III
Distribution of Alcohols in Product

| Experiment No. | 12 | 13 | 14 | 15 | 16 | 17 | 18 | 19 | 20 | 21 | 22 |
|---|---|---|---|---|---|---|---|---|---|---|---|
| $M_b$ | $Pd_{0.05}$ | $Cr_{0.3}$ | $La_{0.15}$ | $Ce_{0.3}$ | $Ti_{0.15}$ | $Zn_{0.15}$ | $Al_{0.15}$ | $Pd_{0.05}$ | $U_{0.2}$ | $Cr_{0.15}$ | $Ce_{0.15}$ |
| Liquid analysis, Wt. % | | | | | | | | | | | |
| methanol | 67.1 | 83.5 | 83.4 | 75.8 | 84.4 | 77.7 | 84.0 | 64.6 | 92.2 | 78.8 | 77.8 |
| ethanol | 3.5 | 1.7 | 1.8 | 1.1 | 1.2 | 2.7 | 2.4 | 3.1 | 1.9 | 1.8 | 1.5 |
| acetone | 0.4 | 0.5 | 0.5 | — | 0.6 | 0.3 | 0.4 | 0.7 | — | 0.6 | 0.6 |
| iso-$C_3$OH | 1.0 | 0.5 | 0.6 | 1.3 | 0.4 | 0.7 | 0.7 | 0.9 | 0.1 | 0.7 | 0.7 |
| n-$C_3$OH | 4.7 | 1.4 | 1.5 | 0.9 | 0.9 | 1.6 | 2.1 | 3.9 | 0.4 | 2.3 | 1.6 |
| sec-$C_4$OH | 0.7 | 1.1 | 0.7 | 1.5 | 1.1 | 0.4 | 0.6 | 1.3 | — | 0.9 | 1.1 |
| iso-$C_4$OH | 12.2 | 6.8 | 7.0 | 13.6 | 8.4 | 7.3 | 6.4 | 15.2 | 0.5 | 10.6 | 10.9 |
| n-$C_4$OH | 2.2 | 1.3 | 1.3 | 1.8 | 1.3 | 1.4 | 0.7 | 2.6 | 0.04 | 1.3 | 1.7 |
| iso-$C_5$OH | 0.7 | 0.1 | 0.2 | — | trace | 0.5 | 0.4 | 0.5 | 0.06 | 0.8 | 0.4 |
| n-$C_5$OH | 2.8 | 0.7 | 1.2 | 1.7 | trace | 1.7 | 1.1 | 2.4 | 0.07 | 1.3 | 1.2 |
| iso-$C_6$OH | 0.5 | 0.2 | 0.2 | 0.1 | 0.8 | 0.3 | 0.1 | 0.5 | — | 0.2 | 0.3 |
| n-$C_6$OH | 1.8 | 0.7 | 0.8 | 1.2 | 0.5 | 1.2 | 0.3 | 2.1 | 0.1 | 0.9 | 1.1 |
| -$C_6$AlC | 2.4 | 1.5 | 0.8 | 1.1 | 0.4 | 4.2 | 0.8 | 2.2 | 4.6 | 0.2 | 1.1 |

TABLE III (Cont'd)

| Experiment No. | 12 | 13 | 14 | 15 | 16 | 17 | 18 | 19 | 20 | 21 | 22 |
|---|---|---|---|---|---|---|---|---|---|---|---|
| $M_b$ | $Pd_{0.05}$ | $Cr_{0.3}$ | $La_{0.15}$ | $Ce_{0.3}$ | $Ti_{0.15}$ | $Zn_{0.15}$ | $Al_{0.15}$ | $Pd_{0.05}$ | $U_{0.2}$ | $Cr_{0.15}$ | $Ce_{0.15}$ |
| Weight %, MeOH free basis | | | | | | | | | | | |
| ethanol | 10.64 | 10.3 | 10.84 | 4.55 | 7.69 | 11.80 | 15.0 | 8.76 | 24.5 | 8.50 | 6.76 |
| acetone | 1.22 | 3.03 | 3.01 | — | 3.84 | 1.32 | 2.50 | 1.98 | — | 2.83 | 2.70 |
| iso-$C_3$OH | 3.04 | 3.03 | 3.62 | 5.37 | 2.57 | 3.09 | 4.38 | 2.54 | 1.3 | 3.31 | 3.15 |
| n-$C_3$OH | 14.29 | 8.48 | 9.03 | 3.72 | 5.77 | 7.05 | 13.13 | 11.03 | 0.4 | 10.85 | 7.21 |
| sec-$C_4$OH | 2.13 | 6.67 | 4.22 | 6.20 | 7.04 | 1.77 | 3.75 | 3.67 | — | 4.25 | 4.96 |
| iso-$C_4$OH | 37.10 | 41.20 | 42.20 | 56.20 | 53.90 | 32.20 | 40.00 | 43.00 | 6.4 | 50.00 | 49.20 |
| n-$C_4$OH | 6.68 | 7.88 | 7.83 | 7.43 | 8.33 | 6.17 | 4.38 | 7.34 | 0.5 | 6.07 | 7.66 |
| iso-$C_5$OH | 2.13 | 0.61 | 1.20 | — | trace | 2.22 | 2.50 | 1.41 | 0.8 | 3.77 | 1.80 |
| n-$C_5$OH | 8.52 | 4.25 | 7.22 | 7.02 | trace | 7.48 | 6.88 | 6.78 | 0.9 | 6.13 | 5.40 |
| iso-$C_6$OH | 1.52 | 1.21 | 1.20 | 0.41 | 5.12 | 1.32 | 0.63 | 1.41 | — | 0.94 | 1.35 |
| n-$C_6$OH | 5.48 | 4.25 | 4.82 | 4.95 | 3.21 | 5.28 | 1.88 | 5.93 | 1.3 | 4.25 | 4.96 |
| -$C_6$AlC | 7.29 | 9.10 | 4.82 | 4.54 | 2.57 | 18.50 | 5.00 | 6.22 | 59.1 | 0.94 | 4.96 |
| Iso/normal ratio | | | | | | | | | | | |
| $C_3$ | .213 | .357 | .401 | 1.44 | .445 | .438 | .333 | .230 | 3.25 | .305 | .437 |
| $C_4$ | 5.55 | 6.33 | 5.40 | 7.57 | 6.48 | 5.22 | 9.13 | 12.8 | 5.85 | 8.23 | 6.43 |
| $C_5$ | 0.250 | 0.144 | 0.166 | | — | 0.297 | 0.363 | 0.208 | 0.89 | 0.615 | 0.333 |
| $C_6$ | 0.277 | 0.285 | 0.249 | 0.083 | 1.59 | 0.250 | 0.335 | 0.238 | — | 0.221 | 0.272 |
| overall | 0.917 | 1.101 | .097 | 1.830 | 1.922 | 0.9 | 1.055 | 1.111 | 2.75 | 1.449 | 1.502 |

TABLE IV

| Experiment No. | 23 | 24 | 25 | 26 | 27 | 28 | 29 | 30 | 31 | 32 | 33 |
|---|---|---|---|---|---|---|---|---|---|---|---|
| $M_b$ | $Ca_{0.15}$ | $Mo_{0.15}$ | $Rh_{0.025}$ | $Ce_{0.5}$ | $Ce_{0.75}$ | $Mn_{0.15}$ | $V_{0.15}$ | $Ru_{0.05}$ | $Rh_{0.05}$ | $Re_{0.05}$ | $Pt_{0.05}$ |
| Liquid analysis, weight % | | | | | | | | | | | |
| methanol | 84.40 | 86.58 | 87.78 | 86.23 | 89.02 | 85.52 | 91.30 | 92.02 | 90.95 | 91.09 | 88.40 |
| ethanol | 1.44 | 1.59 | 1.69 | 1.55 | 2.57 | 1.83 | 1.09 | 2.95 | 2.11 | — | 2.45 |
| acetone | 0.22 | — | 0.12 | 0.25 | 0.21 | 0.38 | 0.41 | 0.96 | — | 0.84 | 0.18 |
| iso-$C_3OH$ | 0.67 | 0.64 | 0.47 | 0.46 | 0.53 | 0.63 | 0.44 | — | 0.43 | 1.38 | 0.51 |
| n-$C_3OH$ | 1.26 | 1.42 | 1.58 | 1.96 | 2.45 | 1.43 | 0.82 | 1.11 | 1.36 | 0.25 | 2.01 |
| sec-$C_4OH$ | 0.50 | 0.31 | 0.16 | 0.33 | 0.21 | 0.53 | 0.59 | 0.17 | 0.18 | 0.64 | 0.21 |
| iso-$C_4OH$ | 6.32 | 6.31 | 5.64 | 6.60 | 3.07 | 6.05 | 4.41 | 1.29 | 3.47 | 0.49 | 3.74 |
| n-$C_4OH$ | 0.87 | 1.07 | 0.87 | 0.99 | 0.58 | 1.38 | 0.18 | — | 0.49 | 1.34 | 0.73 |
| iso-$C_5OH$ | 0.37 | 0.22 | 0.05 | — | 0.07 | — | 0.15 | 0.18 | 0.06 | — | 0.18 |
| n-$C_5OH$ | 0.70 | 0.94 | 0.77 | 0.85 | 0.42 | 0.97 | 0.17 | 0.09 | 0.43 | 1.13 | 0.67 |
| iso-$C_6OH$ | 0.23 | 0.20 | 0.09 | 0.10 | 0.02 | — | 0.12 | 0.10 | 0.15 | 0.62 | 0.13 |
| n-$C_6OH$ | 0.32 | 0.57 | 0.52 | 0.50 | 0.22 | 0.56 | 0.05 | 0.07 | 0.23 | 1.34 | 0.36 |
| $C_6AlC$ | 2.70 | 0.15 | 0.26 | 0.19 | 0.63 | 0.73 | 0.27 | 1.07 | 0.15 | 0.90 | 0.43 |
| Weight %, MeOH free basis | | | | | | | | | | | |
| ethanol | 9.23 | 11.85 | 13.83 | 11.26 | 23.41 | 12.64 | 12.53 | 36.97 | 23.31 | — | 21.12 |
| acetone | 1.41 | — | 0.98 | 1.82 | 1.91 | 2.62 | 4.71 | 12.03 | — | 9.43 | 1.55 |
| iso-$C_3OH$ | 4.29 | 4.77 | 3.85 | 3.34 | 4.83 | 4.35 | 5.06 | — | 4.75 | 15.49 | 4.40 |
| n-$C_3OH$ | 8.08 | 10.58 | 12.93 | 14.23 | 22.31 | 9.88 | 9.43 | 13.91 | 15.03 | 2.81 | 17.33 |

TABLE IV (Cont'd)

| Experiment No. | 23 | 24 | 25 | 26 | 27 | 28 | 29 | 30 | 31 | 32 | 33 |
|---|---|---|---|---|---|---|---|---|---|---|---|
| $M_b$ | $Ca_{0.15}$ | $Mo_{0.15}$ | $Rh_{0.025}$ | $Ce_{0.5}$ | $Ce_{0.75}$ | $Mn_{0.15}$ | $V_{0.15}$ | $Ru_{0.05}$ | $Rh_{0.05}$ | $Re_{0.05}$ | $Pt_{0.05}$ |
| Weight %, MeOH free basis (cont.) | | | | | | | | | | | |
| sec-$C_4OH$ | 3.21 | 2.31 | 1.31 | 2.40 | 1.91 | 3.66 | 6.78 | 2.13 | 1.99 | 7.18 | 1.81 |
| iso-$C_4OH$ | 40.51 | 47.02 | 46.15 | 47.93 | 27.96 | 41.78 | 50.69 | 16.16 | 38.34 | 5.58 | 32.24 |
| n-$C_4OH$ | 5.58 | 7.97 | 7.12 | 3.52 | 5.28 | 9.53 | 2.07 | — | 5.51 | 15.04 | 6.29 |
| iso-$C_5OH$ | 2.37 | 1.67 | 0.41 | — | 0.64 | — | 1.72 | 2.26 | 0.66 | — | 1.55 |
| n-$C_5OH$ | 4.49 | 7.00 | 6.30 | 6.17 | 3.82 | 6.70 | 1.95 | 1.13 | 4.75 | 12.68 | 5.78 |
| iso-$C_6OH$ | 1.47 | 1.49 | 0.74 | 0.73 | 0.18 | — | 1.38 | 1.25 | 1.66 | 6.96 | 1.12 |
| n-$C_6OH$ | 2.05 | 4.25 | 4.26 | 3.63 | 2.00 | 3.87 | 0.57 | 0.88 | 2.54 | 15.04 | 3.10 |
| $C_6AlC$ | 17.31 | 1.11 | 2.13 | 1.38 | 5.74 | 5.04 | 3.10 | 13.41 | 1.66 | 10.10 | 3.71 |
| iso/normal ratio | | | | | | | | | | | |
| $C_3$ | 0.531 | 0.451 | 0.298 | 0.235 | 0.216 | 0.440 | 0.537 | 0 | 0.316 | 5.51 | 0.254 |
| $C_4$ | 4.61 | 4.57 | 5.47 | 8.10 | 3.89 | 3.17 | 5.73 | 7.59 | 5.11 | 0.248 | 3.98 |
| $C_5$ | 0.528 | 0.239 | 0.065 | — | 0.167 | — | 0.882 | 2.97 | 0.139 | 0 | 0.268 |
| $C_6$ | 0.717 | 0.351 | 0.174 | 0.201 | 0.090 | — | 2.42 | 1.42 | 0.654 | 0.463 | 0.361 |
| overall | 1.490 | 1.250 | 1.118 | 1.262 | 0.572 | 0.997 | 1.766 | 0.358 | 0.855 | 0.530 | 0.709 |

From the foregoing, it can be seen that alcohol mixtures having a novel distribution of alcohols as described above are produced by a very simple and straightforward procedure. Each of these alcohol mixtures can be directly mixed with gasoline to provide novel alcohol/gasoline compositions, or, if desired, those alcohol mixtures containing more than 85% methanol can be fractionally distilled prior to admixing with gasoline to remove excess methanol.

Although only a few embodiments of the present invention have been described above, many modifications can be made without departing from the spirit and scope of the invention. All such modifications are intended to be included within the scope of the present invention which is to be limited only by the following claims.

**Claims**

1. A process for producing an alcohol mixture, by contacting a gaseous reactant containing carbon monoxide and hydrogen with a metal, oxide complex catalyst at a pressure of no more than 103 bar (1.500 psi) and at a temperature of 200 to 425°C, characterized in that a catalyst is used having the empirical formula:

$$Cu_aThM_bA_cO_x$$

wherein
M is one or more of Ca, Mo, Rh, Mn, Pt, Ce, Cr, Zn, Al, Ti, La, V, U, Re, Ru and Pd;
A is an alkali metal; and
wherein
a is 0.5 to 2.5;
b is 0.01 to 1.0;
c is 0.05 to 0.9; and
x is a number such that the valence requirements of the other elements for oxygen is satisfied,
said catalyst being prepared by:

(1) adding an alkali metal carbonate or hydroxide to a solution containing decomposable salts of thorium, copper and "M" to raise the pH of the solution to at least 7.5 and form a precipitate,
(2) heating the precipitate in its mother liquor until change in color of said precipitate has ceased,
(3) neutralizing the mother liquor,
(4) recovering the precipitate, and
(5) calcining the precipitate in the presence of an oxygen-containing gas.

2. The process according to claim 1, characterized in that, if a carbonate is added in step (1) of the catalyst preparation process, said process further comprises:

(6) contacting said catalyst with a reducing gas while maintaining the temperature of said catalyst below the temperature at which significant sintering occurs.

3. The process according to claim 1 or 2, characterized in that the composition being heated in step (2) of the catalyst preparation process is maintained at a temperature of 80 to 95°C during the heating operation step (2).

4. The process according to any of claims 1 to 3, characterized in that the $H_2$ to CO ratio in said gaseous reactant is from 0.6 to 6.

5. The process according to claim 4 wherein said gaseous reactant is synthesis gas.

6. The process according to any of claims 1 to 5, characterized in that said reaction is carried out in fixed-bed mode.

7. The catalyst according to claims 1 to 6 comprising an oxide complex of the empirical formula:

$$Cu_aThM_bA_cO_x$$

wherein
M is one or more of Ca, Mo, Rh, Mn, Pt, Ce, Cr, Zn, Al, Ti, La, V, U, Re, Ru or Pd;
A is an alkali metal; and
wherein
a is 0.5 to 2.5;
b is 0.01 to 1.0;
c is 0.05 to 0.9; and
x is a number such that the valence requirements of the other elements for oxygen is satisfied, said catalyst being prepared by:

(1) adding an alkali metal carbonate or hydroxide to a solution containing decomposable salts of thorium, copper and "M" to raise the pH of the solution to at least 7.5 and form a precipitate,
(2) heating the precipitate in its mother liquor until change in color of said precipitate has ceased,
(3) neutralizing the mother liquor,
(4) recovering the precipitate, and
(5) calcining the precipitate in the presence of an oxygen-containing gas.

8. The catalyst according to claim 7, characterized in that a carbonate is added in step (1) of the catalyst preparation process and said process further comprises:

(6) contacting said catalyst with a reducing gas while maintaining the temperature of said catalyst below the temperature at which significant sintering occurs.

9. The catalyst according to claims 7 or 8, characterized in that the composition being heated in step (2) is maintained at a temperature of 80 to 95°C during the heating operation of step (2).

10. The catalyst according to any of claims 7 to 9, characterized in that M is Cr, Zn, Al, Ti, La, V and/or Pd.

11. The catalyst according to any of claims 7 to 10, characterized in that A is sodium.

12. A modified gasoline composition containing a homogeneous solution of gasoline and a mixture of alcohols, obtained by the process according to claims 1 to 6 characterized in that said mixture of alcohols comprises at least 40% methanol, the remainder of said mixture comprising higher alcohols, the distribution of said higher alcohols on a methanol-free basis being:

| | |
|---|---|
| $C_2$ | 4—25 weight % |
| $C_3$ | 0.1—25 weight % |
| $C_4$ | 0.5—70 weight % |
| $C_5$ | 0.1—12 weight % |
| $C_6$ | 0.1—10 weight % |

13. The modified gasoline composition according to claim 12, characterized in that said mixture contains no more than 85% methanol.

14. The modified gasoline composition according to claim 13, wherein $C_3$ alcohols are present in the amount of 9 to 25 weight % and $C_4$ alcohols are present in the amount of 40 to 70 weight %.

15. The modified gasoline composition according to any of claims 12 to 14, characterized in that the gasoline component of said gasoline contains both paraffinic and aromatic fractions.

16. The modified gasoline composition according to any of claims 12 to 14, characterized in that the gasoline component of said modified gasoline contains paraffins only.

**Patentansprüche**

1. Verfahren zur Herstellung eines Alkoholgemischs durch Kontakt einer gasförmigen Reaktionskomponente, die Kohlenmonoxid und Wasserstoff enthält, mit einem Metalloxid-Komplex-Katalysator bei einem Druck von nicht mehr als 103 bar (1500 psi) und bei einer Temperatur von 200 bis 425°C, dadurch gekennzeichnet, daß ein Katalysator verwendet wird, der folgende empirische Formel aufweist:

$$Cu_aThM_bA_cO_x$$

worin

M eines oder mehrere von Ca, Mo, Rh, Mn, Pt, Ce, Cr, Zn, Al, Ti, La, V, U, Re, Ru und Pd ist;

A ein alkalimetall ist; und

worin

a 0,5 bis 2,5 ist;

b 0,01 bis 1,0 ist;

c 0,05 bis 0,9 ist; und

x eine derartige Zahl ist, daß die Wertigkeitserfordernisse der anderen Elemente für Sauerstoff erfüllt werden; wobei der Katalysator hergestellt wird durch

1. Zusatz eines Alkalimetallcarbonats oder -hydroxids zu einer Lösung, die zersetzliche Salze von Thorium, Kupfer und "M" enthält, um den pH-Wert der Lösung auf mindestens 7,5 anzuheben und eine Ausfällung zu bilden,
2. Erwärmen der Ausfällung in ihrer Mutterlauge bis die Farbänderung der Ausfällung beendet ist,
3. Neutralisieren der Mutterlauge,
4. Gewinnen der Ausfällung und
5. Calcinieren der Ausfällung in Anwesenheit eines Sauerstoff enthaltenden Gases.

2. Verfahren nach Anspruch 1, dadurch gekennzeichnet, daß, wenn in der Stufe 1 des Herstellungsverfahrens für den Katalysator ein Carbonat zugesetzt wird, das Verfahren darüberhinaus umfaßt:

6. den Kontakt des Katalysators mit einem reduzierenden Gas, wobei die Temperatur des Katalysators unter der Temperatur gehalten wird, bei der ein beträchtliches Sintern auftritt.

3. Verfahren nach Anspruch 1 oder 2, dadurch gekennzeichnet, daß die in der Stufe 2 des Herstellungsverfahrens für den Katalysator erwärmte Zusammensetzung bei einer Temperatur von 80 bis 95°C während der Erwärmungsverfahrensstufe 2 gehalten wird.

4. Verfahren nach einem der Ansprüche 1 bis 3, dadurch gekennzeichnet, daß das $H_2$ zu CO-Verhältnis in der gasförmigen Reaktionskomponente 0,6 bis 6 beträgt.

5. Verfahren nach Anspruch 4, bei dem die gasförmige Reaktionskomponente Synthese-Gas ist.

6. Verfahren nach eimem der Ansprüche 1 bis 5, dadurch gekennzeichnet, daß die Reaktion in Festbett-Verfahrensweise durchgeführt wird.

7. Katalysator nach einem der Ansprüche 1 bis 6, enthaltend einen Oxidkomplex mit der empirischen Formel

$$Cu_aThM_bA_cO_x$$

worin

M eines oder mehrere von Ca, Mo, Rh, Mn, Pt, Ce, Cr, Zn, Al, Ti, La, V, U, Re, Ru oder Pd ist;

A ein Alkalimetall ist; und

worin

a 0,5 bis 2,5 ist;

b 0,01 bis 1,0 ist;

c 0,05 bis 0,9 ist; und

x eine derartige Zahl ist, daß die Wertigkeitserfordernisse der anderen Elemente für Sauerstoff erfüllt werden, wobei der Katalysator hergestellt wird durch:

1. Zusatz eines Alkalimetallcarbonats oder -hydroxids zu einer Lösung, die zersetzliche Salze von Thorium, Kupfer und "M" enthält, um den pH-Wert der Lösung auf mindestens 7,5 anzuheben und eine Ausfällung zu bilden,
2. Erwärmen der Ausfällung in ihrer Mutterlauge, bis die Farbänderung der Ausfällung beendet ist,
3. Neutralisieren der Mutterlauge,
4. Gewinnen der Ausfällung und
5. Calcinieren der Ausfällung in Anwesenheit eines Sauerstoff enthaltenden Gases.

8. Katalysator nach Anspruch 7, dadurch gekennzeichnet, daß ein Carbonat in der Stufe 1 des Herstellungsverfahrens des Katalysators zugesetzt wird und das Verfahren darüberhinaus umfaßt:

6. den Kontakt des Katalysators mit einem reduzierenden Gas, während die Temperatur des Katalysators unter der Temperatur gehalten wird, bei der ein beträchtliches Sintern auftritt.

9. Katalysator nach Anspruch 7 oder 8, dadurch gekennzeichnet, daß die in der Stufe 2 erwärmte Zusammensetzung bei einer Temperatur von 80 bis 95°C während der Erwärmungsverfahrensstufe 2 gehalten wird.

10. Katalysator nach einem der Ansprüche 7 bis 9, dadurch gekennzeichnet, daß M Cr, Zn, Al, Ti, La, V und/oder Pd ist.

11. Katalysator nach einem der Ansprüche 7 bis 10, dadurch gekennzeichnet, daß A Natrium ist.

12. Modifizierte Benzinzusammensetzung, enthaltend eine homogene Lösung von Benzin und einem Gemisch von Alkoholen, erhalten nach dem Verfahren der Ansprüche 1 bis 6, dadurch gekennzeichnet, daß das Gemisch von Alkoholen mindestens 40% Methanol enthält und der Rest des Gemischs höhere Alkohole enthält, wobei sich die höheren Alkohole auf methanolfreier Basis wie folgt verteilen:

| | |
|---|---|
| $C_2$ | 4—25 Gew.-% |
| $C_3$ | 0,1—25 Gew.-% |
| $C_4$ | 0,5—70 Gew.-% |
| $C_5$ | 0,1—12 Gew.-% |
| $C_6$ | 0,1—10 Gew.-% |

13. Modifizierte Benzinzusammensetzung nach Anspruch 12, dadurch gekennzeichnet, daß das Gemisch nicht mehr als 85% Methanol enthält.

14. Modifizierte Benzinzusammensetzung nach Anspruch 13, worin $C_3$-Alkohole in einer Menge von 9 bis 25 Gew.-% und $C_4$-Alkohole in einer Menge von 40 bis 70 Gew.-% vorhanden sind.

15. Modifizierte Benzinzusammensetzung nach einem der Ansprüche 12 bis 14, dadurch gekennzeichnet, daß die Benzinkomponente des Benzins sowohl paraffinische als auch aromatische Fraktionen enthält.

16. Modifizierte Benzinzusammensetzung nach einem der Ansprüche 12 bis 14, dadurch gekennzeichnet, daß die Benzinkomponente des modifizierten Benzins nur Paraffine enthält.

**Revendications**

1. Procédé pour produire un mélange d'alcohols en mettant en contact un réactif gazeux contenant du monoxyde de carbone et de l'hydrogène avec un catalyseur complexe aux oxydes métalliques sous une pression non-supérieure à 103 bar (1500 psi) et à une température de 200 à 425°C, caractérisé par le fait que l'on utilise un catalyseur ayant la formule empirique:

$$Cu_aThM_bA_cO_x$$

où

M est au moins l'un parmi Ca, Mo, Rh, Mn, Pt, Ce, Cr, Zn, Al, Ti, La, V, U, Re, Ru ou Pd;
A est un métal alcalin; et

où

a vaut 0,5 à 2,5;
b vaut 0,01 à 1,0;
c vaut 0,05 à 0,09; et
x est un nombre satisfaisant, pour l'oxygène, aux exigences de valence des autres éléments, ce catalyseur étant préparé:

(1) en ajoutant un hydroxyde ou un carbonate de métal alcalin à une solution contenant des sels décomposables de thorium, de cuivre et de "M" pour élever le pH de la solution à au moins 7,5 et former un précipité,
(2) en chauffant le précipité dans sa liqueur-mère jusqu'à ce que le changement de couleur du précipité ait cessé,
(3) en neutralisant la liqueur-mère,
(4) en récupérant le précipité, et
(5) en calcinant le précipité en présence d'un gaz contenant de l'oxygène.

2. Procédé selon la revendication 1, caractérisé par le fait que, si l'on ajoute un carbonate à l'étape (1) du procédé de préparation du catalyseur, ce procédé comprend en outre:

(6) la mise en contact dudit catalyseur avec un gaz réducteur, tout en maintenant la température du catalyseur en-dessous de la température à laquelle se produit un frittage important.

3. Procédé selon l'une des revendications 1 ou 2, caractérisé par le fait que la composition chauffée à l'étape (2) du procédé de préparation de catalyseur est maintenue à une température de 80 à 95°C pendant l'étape de chauffage (2).

4. Procédé selon l'une des revendications 1 à 3, caractérisé par le fait que le rapport du $H_2$ au CO dans le réactif gazeux est compris entre 0,6 et 6.

5. Procédé selon la revendication 4, dans lequel le réactif gazeux est du gaz de synthèse.

6. Procédé selon l'une des revendications 1 à 5 caractérisé par le fait que la réaction est mise en oeuvre dans un mode à lit fixe.

7. Catalyseur selon l'une des revendications 1 à 6, comprenant un complexe d'oxydes de formule empirique:

$$Cu_aThM_bA_cO_x$$

où

M est au moins l'un parmi Ca, Mo, Rh, Mn, Pt, Ce, Cr, Zn, Al, Ti, La, V, U, Re, Ru ou Pd;

A est un métal alcalin; et

où

a vaut 0,5 à 2,5;

b vaut 0,01 à 1,0;

c vaut 0,05 à 9,9; et

x est un nombre satisfaisant, pour l'oxygène, aux exigences de valence des autres éléments, ce catalyseur étant prépare:

(1) en ajoutant un hydroxyde ou un carbonate de métal alcalin à une solution contenant des sels décomposables de thorium, de cuivre et de "M" pour élever le pH de la solution à au moins 7,5 et former un précipité,
(2) en chauffant le précipité dans sa liqueur-mère jusqu'à ce que le changement de couleur du précipité ait cessé,
(3) en neutralisant la liqueur-mère,
(4) en récupérant le précipité, et
(5) en calcinant le précipité en présence d'un gaz contenant de l'oxygène.

8. Catalyseur selon la revendication 7, caractérisé par le fait qu'on a joute un carbonate à l'étape (1) du procédé de préparation du catalyseur, ce procédé comprenant en outre l'étape consistant à:

(6) à mettre en contact le catalyseur avec un gaz réducteur tout en maintenant la température dudit catalyseur en-dessous de la température à laquelle se produit un frittage important.

9. Catalyseur selon l'une des revendications 7 ou 8, caractérisé par le fait que la composition chauffée à l'étape (2) est maintenue à une température de 80 à 95°C pendant l'opération de chauffage de l'étape (2).

10. Catalyseur selon l'une quelconque des revendications 7 à 9, caractérisé par le fait que M est Cr, Zn, Al, Ti, La, V et/ou Pd.

11. Catalyseur selon l'une quelconque des revendications 7 à 10, caractérisé par le fait que A est le sodium.

12. Composition d'essence modifiée contenant une solution homogène d'essence et d'un mélange d'alcools obtenu par le procédé selon l'une des revendications 1 à 6, caractérisée par le fait que ledit mélange d'alcools se compose d'au moins 40% de méthanol, le reste dudit mélange étant composé d'alcools supérieurs, la distribution de ces alcools supérieurs, sur une base sans méthanol, étant:

| | |
|---|---|
| $C_2$ | 4—25% en poids |
| $C_3$ | 0,1—25% en poids |
| $C_4$ | 0,5—70% en poids |
| $C_5$ | 0,1—12% en poids |
| $C_6$ | 0,1—10% en poids |

13. Composition d'essence modifiée selon la revendication 12, caractérisée par le fait que ledit mélange ne contient pas plus de 85% de méthanol.

14. Composition d'essence modifiée selon la revendication 13, où des alcools en $C_3$ sont présents en une quantité de 9 à 25% en poids, et des alcools en $C_4$ sont présents en une quantité de 40 à 70% en poids.

15. Composition d'essence modifiée selon l'une des revendications 12 à 14, caractérisé par le fait que le constituant essence de l'essence contient tant des fractions paraffiniques que des fractions aromatiques.

16. Composition d'essence modifiée selon l'une quelconque des revendications 12 à 14, caractérisé par le fait que le constituant essence de l'essence modifiée ne contient que des paraffiniques.